# EUROPEAN PATENT SPECIFICATION

(11) **EP 1 615 652 B1**
(45) Date of publication and mention of the grant of the patent: **25.04.2007**
(21) Application number: 04726957.6
(22) Date of filing: 13.04.2004
(51) Int. Cl.: A61K 38/17, A61P 11/00

(54) **TREATMENT OF SEVERE ACUTE RESPIRATORY SYNDROME (SARS)**
BEHANDLUNG DES SCHWEREN AKUTEN ATEMWEGSSYNDROMS (SARS)
TRAITEMENT DU SARS

(30) Priority: 09.04.2003 DK 200300549; 10.05.2003 DK 200300714
(43) Date of publication of application: 18.01.2006
(73) Proprietor: Natimmune A/S, 2100 Kobenhavn 0 (DK); Aarhus Universitet, 8000 Aarhus C (DK)
(72) Inventor: KONGERSLEV, Leif, 3460 Birkerod (DK); WEILGUNY, Dietmar, 2830 Virum (DK); MATTHIESEN, Finn, 2700 Bronshoj (DK); JENSENIUS, Jens, Christian, 5230 Odense M (DK)
(74) Representative: HOEIBERG A/S
(86) International application number: PCT/DK2004/000266
(87) International publication number: WO 2004/089394

(56) References cited:
- DEMIRKIRAN O ET AL: "Severe Acute Respiratory Syndrome" SENDROM 01 APR 2003 TURKEY, vol. 15, no. 4, 1 April 2003 (2003-04-01), pages 88-95, XP009050189 ISSN: 1016-5134
- CYRANOSKI D: "China joins investigation of mystery pneumonia" NATURE 03 APR 2003 UNITED KINGDOM, vol. 422, no. 6931, 3 April 2003 (2003-04-03), page 459, XP009050188 ISSN: 0028-0836
- DATABASE WPI Section Ch, Week 200334 Derwent Publications Ltd., London, GB; Class A97, AN 2003-363012 XP002335844 & WO 03/018617 A1 (FUSO PHARM IND LTD) 6 March 2003 (2003-03-06)
- HSUEH PO-REN ET AL: "Severe acute respiratory syndrome (SARS) - An emerging infection of the 21st century" JOURNAL OF THE FORMOSAN MEDICAL ASSOCIATION, vol. 102, no. 12, December 2003 (2003-12), pages 825-839, XP009050186 ISSN: 0929-6646
- LIN L ET AL: "Treating severe acute respiratory syndrome with integrated Chinese and Western medicine - A report on 103 hospitalised cases at the Second Affiliated Hospital of Guangzhou University of Chinese Medicine, China" JOURNAL OF CHINESE MEDICINE 2003 UNITED KINGDOM, no. 72, 2003, pages 5-10, XP009050196 ISSN: 0143-8042

## Description

The present invention pertains to the use of subunits and oligomers of collectins and/or ficolins, such as mannan-binding lectin (MBL) for the manufacture of a medicament for the prophylactic and/or curative treatment of Severe Acute Respiratory Syndrome (SARS) in an individual.

SARS infection presents the symptoms of high fever, dry cough, myalgin (muscle soreness) and sore throat. Most individuals suffering from SARS develop breathing difficulties eventually requiring ventilator support, and severe thrombocytopenia. 5-10 percent of individuals suffering from SARS will eventually die due to the disease.

The cause of SARS is not yet known. It has been speculated that SARS may be caused by a virus and among these, coronaviruses and paramyxoviruses have been mentioned.

Symptoms of SARS seem to start 2-14 days after exposure.

### Summary of the Invention

By the present invention treatment and/or prophylaxis of Severe Acute Respiratory Syndrome using collectins and/or ficolins is suggested.

Collectins all exhibit the following architecture: they have an N-terminal cysteine-rich region that appears to form inter-chain disulfide bonds, followed by a collagen-like region, an α-helical coiled-coil region and finally a C-type lectin domain which is the pattern-recognizing region and is referred to as the carbohydrate recognition domain (CRD). The name collectin is derived from the presence of both collagen and lectin domains. The α-helical coiled-coil region initiates trimerisation of the individual polypetides to form collagen triple coils, thereby generating collectin subunits each consisting of 3 individual polypeptides, whereas the N-terminal region mediates formation of oligomers of subunits. Different collectins exhibit distinctive higher order structures, typically either tetramers of subunits or hexamers of subunits. The grouping of large numbers of binding domains allows collectins to bind with high avidity to microbial cell walls, despite a relatively low intrinsic affinity of each individual CRD for carbohydrates.

C-type CRDs are found in proteins with a widespread occurrence, both in phylogenetic and functional perspective. The different CRDs of the different collectins enable them to recognise a range of distinct microbial surface components exposed on different microorganisms. The terminal CRDs are distributed in such a way that all three domain target surfaces that present binding sites has a spacing of approximately 53 A (Sheriff *et al.,* 1994; Weis & Drickamer, 1994). This property of 'pattern recognition' may contribute further to the selectively binding of microbial surfaces. The collagenous region or possibly the N-terminal tails of the collectins, are recognised by specific receptors on phagocytes, and is the binding site for associated proteases that are activated to initiate the complement cascade upon binding of the CRD domain to a target.

Ficolins, like MBL, are lectins that contain a collagen-like domain. Unlike MBL, however, they have a fibrinogen-like domain, which is similar to fibrinogen β- and γ-chains. Ficolins also forms oligomers of structural subunits, each of which is composed of three identical 35 kDa polypeptides. Each subunit is composed of an amino-terminal, cysteine-rich region; a collagen-like domain that consists of tandem repeats of Gly-Xaa-Yaa triplet sequences (where Xaa and Yaa represent any amino acid); a neck region; and a fibrinogen-like domain. The oligomers of ficolins comprises two or more subunits, especially a tetrameric form of ficolin has been observed.

Some of the ficolins triggers the activation of the complement system substantially in similar way as done by MBL. This triggering of the complement system results in the activation of novel serine proteases (MASPs) as described above.

The fibrinogen-like domain of several lectins has a similar function to the CRD of C-type lectins including MBL, and hereby function as pattern-recognition receptors to discriminate pathogens from self.

Serum ficolins have a common binding specificity for GlcNAc (N-acetylglucosamine), elastin or GalNAc (N-acetyl-galactosamine). The fibrinogen-like domain is responsible for the carbohydrate binding. In human serum, two types of ficolin, known as L-ficolin (P35, ficolin L, ficolin 2 or hucolin) and H-ficolin (Hakata antigen, ficolin 3 or thermolabile b2-macroglycoprotein), have been identified, and both of them have lectin activity. L-ficolin recognises GlcNAc and H-ficolin recognises GalNAc. Another ficolin known as M-ficolin (P35-related protein, Ficolin 1 or Ficolin A) is not considered to be a serum protein and is found in leucocytes and in the lungs. L-ficolin and H-ficolin activate the lectin-complement pathway in association with MASPs. M-Ficolin, L-ficolin and H-ficolin has calcium-independent lectin activity.

Mannan-binding lectin (MBL), synonymous to mannose-binding lectin, mannan-binding protein or mannose-binding protein (MBP), belongs to a subgroup of C-type lectins, termed collectins, since these soluble proteins are composed of subunits presenting three CRDs attached to a collagenous stalk². MBL interact with carbohydrates presented by a wide range of micro-organisms and accumulating evidence shows that it plays an important role in the innate immune defence³. When bound to carbohydrate MBL is able to activate the complement system.

The complement system may be activated via three different pathways: the classical pathway, the alternative pathway, and the newly described third pathway, the mannan-binding lectin (MBL) pathway which is initiated by the binding of MBL to carbohydrates presented by micro-organisms. The components of the alternative pathway and of the MBL pathway are parts of the innate immune defence, also termed the natural or the non-clonal, immune defence, while the classical pathway involves cooperation with antibodies of the specific immune defence⁴.

The human MBL protein is composed of up to 18 identical 32 kDa polypeptide chains²⁷, each comprising a short N-terminal segment of 21 amino acids including three cysteine residues, followed by 7 repeats of the collagenous motif Gly-X-Y interrupted by a Gln residues followed by another 12 Gly-X-Y repeats. A small 34 residue 'neck-region' joins the C-terminal Ca²⁺-dependent lectin domain of 93 amino acids with the collagenous part of the molecule²⁸.

The collagenous regions of the three polypeptide chains combine to form a subunit which is stabilised covalently by disulphide bridges. Individual subunits are joined by disulphide bridges as well as by non-covalently interactions²⁷.

The concentration of MBL in human serum is largely genetically determined, but reportedly increases up to threefold during acute phase reactions⁸. Three mutations causing structural alterations and two mutations in the promotor region are associated with MBL deficiency⁹.

A wide range of oligosaccharides can bind to MBL. As the target sugars are not normally exposed on mammalian cell surfaces at high densities, MBL does not usually recognize self-determinants, but is particularly well suited to interactions with microbial cell surfaces presenting repetitive carbohydrate determinants.

Thus, the invention features the use of MBL, purified from natural sources or from material produced by recombinant technologies, or by any other suitable MBL-producing cell line, for the prophylaxis and/or treatment of SARS. The MBL may be given before or after start of the medical treatment and for any duration of time deemed suitable.

MBL is believed to exert its anti-SARS activity mainly through its opsonizing activity (preparation of microorganisms for phagocytosis). This activity is dependent on activation of complement after binding of MBL to the microbial surface and deposition of C4b and C3b on the microorganism. MBL can also promote the direct complement-mediated killing of the microorganism through the activation of the terminal lytic pathway of complement and insertion of the membrane attack complex (MAC) in the membrane. This mechanism is considered of minor importance.

It is possible according to the invention to treat SARS prophylactically. By prophylactic treatment with MBL it is possible to prevent subsequent SARS or to reduce the risk of the individual contracting SARS.

In another aspect the present invention is related to the use of a composition comprising at least one mannan-binding lectin (MBL) subunit, or at least one oligomer comprising the at least one mannan-binding lectin (MBL) subunit, in the manufacture of a medicament for prophylactic, ameliorating or curative treatment of SARS in an individual initially having low plasma levels of MBL, such as plasma levels of about 0 mg/ml, or plasma levels in excess of 10 ng/. In particular the individual may be genetically disposed to an MBL deficiency or have acquired an MBL deficiency leading to an increased risk of suffering from infections. Accordingly, the invention also concerns treatment of SARS in individuals suffering from a mannan-binding lectin (MBL) deficiency including any deficiency in the production of MBL and/or function of MBL, in particular however, individuals who have or are suspected to have SARS.

In yet another aspect there is provided a method for estimating the probability of the occurrence of any severe outcome of SARS in an individual, said method comprising the step of measuring the concentration of MBL in plasma or serum obtained from the individual, and estimating the probability on the basis of the measured concentration.

Also, by genotyping the individuals in question it is possible to estimate the probability.

### Detailed Description of the Invention

SARS may be prevented and/or treated in individuals independent on their serum collectin and/or ficolin level, such as MBL level.

The collectin according to the invention may be any collectin capable of preventing or treating SARS in an individual.

Accordingly, the collectin may be selected from the group consisting of MBL (mannose-binding lectin), SP-A (lung surfactant protein A), SP-D (lung surfactant protein D), BK (or BC, bovine conglutinin), CL-L1 (Ohtani et al. 1999, Molecular cloning of a novel human collectin from liver (CL-L1), J. Biol. Chem. 274:13681-89), CL-P1 (Ohtani et al. 2001. The membrane-type collectin CL-P1 is a scavenger receptor on vascular endothelial cells. J. Biol. Chem. 276:44222-28), and CL-43 (collectin-43). Most preferably the collectin is MBL. (Holmskov et al. 2003, Annu Rev. Immunol. 21:547-78).

In a particular preferred embodiment the collectin has one of the sequences listed below with reference to their database and accession No.

### Collectins

1: Q9NPY3
   Complement component C1q receptor precursor (Complement component 1, q subcomponent, receptor 1) (C1qRp) (C1qR(p)) (C1q/MBUSPA receptor) (CD93 antigen) (CDw93)
   gi|21759074|sp|Q9NPY3|CD93_HUMAN[21759074]
2: BAC05523
   collectin placenta 1 [Mus musculus]
   gi|21901969|dbj|BAC05523.1|[21901969]
3: AAM34743
   46-kDa collectin precursor [Bos taurus]
   gi|21105687|gb|AAM34743.1|AF509590_1[21105687]
4: AAM34742
   46-kDa collectin precursor [Bos taurus]
   gi|21105685|gb|AAM34742.1|AF509589_1[21105685]
5: XP_139613
   similar to collectin sub-family member 10; collectin liver 1; collectin 34 [Mus musculus]
   gi|20903807|ref|XP_139613.1|[20903807]
6: XP_123211
   similar to collectin sub-family member 12 [Mus musculus]
   gi|20876566|ref|XP_123211.1|[20876566]
7: NP_571645
   mannose binding-like lectin [Danio rerio]
   gi|18858997|ref|NP_571645.1|[18858997]
8: NP_569057
   collectin sub-family member 12, isoform I; scavenger receptor with C-type lectin; collectin placenta 1 [Homo sapiens]
   gi|18641360|ref|NP_569057.1|[18641360]
9:NP_110408
   collectin sub-family member 12, isoform II; scavenger receptor with C-type lectin; collectin placenta 1 [Homo sapiens]
   gil|18641358|ref|NP_110408.2|[18641358]
10: NP_569716
   collectin sub-family member 12 [Mus musculus]
   gi|18485494|ref|NP_569716.1|[18485494]
11: AAL51856
   43kDa collectin precursor [Bos taurus]
   gi|18252111|gb|AAL61856.1|[18252111]
12: AAL61855
   43kDa collectin precursor [Bos taurus]
   gi|18252109|gb|AAL61855.1|[18252109]
13: BAB22581
   data source:SPTR, source key:Q9Y6Z7, evidence:ISS~homolog to COLLECTIN 34-putative [Mus musculus]
   gi|12833584|dbj|BAB22581.1|[12833584]
14: NP_034905
   mannose binding lectin, liver (A) [Mus musculus]
   gi|6754654|ref|NP_034905.1|[6754654]
15:NP_034906
   mannose binding lectin, serum (C) [Mus musculus]
   gi|6754656|ref|NP_034906.1|[6754656]
16: NP_006429
   collectin sub-family member 10; collectin liver 1; collectin 34 [Homo sapiens]
   gi[5453619|ref|NP_006429.1|[5453619]
17: BAB72147
   collectin placenta 1 [Homo sapiens]
   gi|17026101|dbj|BAB72147.1|[17026101]
18: AAF63470
   mannose binding-like lectin precursor [Carassius auratus]
   gi|7542474|gb|AAF63470.1|AF227739_1[7542474]
19:AAF63469
   mannose binding-like lectin precursor [Danio rerio]
   gi|7542472|gb|AAF63469.1|AF227738_1[7542472]
20: AAF63468
   mannose binding-like lectin precursor [Cyprinus carpio]
   gi|7542470|gb|AAF63468.1|AF227737_1[7542470]
21: AAK97540
   surfactant protein A precursor [Gallus gallus]
   gi|15420996|gb|AAK97540.1|AF411083_1[15420996]
22: LNMSMC
   mannose-binding lectin C precursor - mouse
   gi|7428747|pir||LNMSMC[7428747]
23: LNMSMA
   mannose-binding lectin A precursor - mouse
   gi|625320|pir||LNMSMA[625320]
24: JN0450
   conglutinin precursor - bovine
   gi|346501|pir||JN0450[346501]
25:A57250
   mannan-binding protein - chicken (fragment)
   gi|1362725|pir||A57250[1362725]
26: A53570
   collectin-43 - bovine
   gi|1083017|pir||A53570[1083017]
27: AAF28384
   lung surfactant protein A [Sus scrofa]
   gi|6782434|gb|AAF28384.1|AF133668_1[6782434]
28: AAF22145
   lung surfactant protein D precursor; SPD; SP-D; CP4 [Sus scrofa]
   gi|6760482|gb|AAF22145.2|AF132496_1[6760482]
29:P41317
   MANNOSE-BINDING PROTEIN C PRECURSOR (MBP-C) (MANNAN-BINDING PROTEIN)
   (RA-REACTIVE FACTOR P28A SUBUNIT) (RARF/P28A)
   gi|1346477|sp|P41317|MABC_MOUSE[1346477]
30:P39039
   MANNOSE-BINDING PROTEIN A PRECURSOR (MBP-A) (MANNAN-BINDING PROTEIN)
   (RA-REACTIVE FACTOR POLYSACCHARIDE-BINDING COMPONENT P28B POLYPEPTIDE) (RARF
   P28B)
   gi|729972|sp|P39039|MABA_MOUSE[729972]
31: P42916
   COLLECTIN-43 (CL-43)
   gi|1168967|sp|P42916|CL43_BOVIN[1168967]
32: CAB56155
   DMBT1/8kb.2 protein [Homo sapiens]
   gi|5912464|emb|CAB56155.1|[5912464]
33: BAA81747
   collectin 34 [Homo sapiens]
   gi|5162875|dbj|BAA81747.1|[5162875]
34: AAB94071
   mannan-binding lectin; collectin [Gallus gallus]
   gi|2736145|gb|AAB94071.1|[2736145]
35: AAB36019
   mannan-binding protein, MBP=lectin {N-terminal} [chickens, serum, Peptide Partial, 30 aa] [Gallus gallus]
   gi|1311692|gb|AAB36019.1|[1311692]
36: AAB27504
   conglutinin (N) {N-terminal} [cattle, Peptide Partial, 60 aa] [Bos taurus]
   gi|386660|gb|AAB27504.1|[386660]
37: CAA53511
   collectin-43 [Bos taurus]
   gi|499385|emb|CAA53511.1|[499385]
38:AAA82010
   mannose-binding protein C [Mus musculus]
   gi|773288|gb|AAA82010.1|[773288]
39: AAA82009
   mannose-binding protein A [Mus musculus]
   gi|773280|gb|AAA82009.1|[773280]

### Lung surfactant protein

1: 1KMRA
   Chain A, Solution Nmr Structure Of Surfactant Protein B (11-25) (Sp- B11-25)
   gi|22219056|pdb|1KMR|A[22219056]
2:P50404
   Pulmonary surfactant-associated protein D precursor (SP-D) (PSP-D)
   gi|1709879|sp|P50404|PSPD_MOUSE[1709879]
3: P06908
   Pulmonary surfactant-associated protein A precursor (SP-A) (PSP-A) (PSAP)
   gi|1172693|sp|P06908|PSPA_CANFA[1172693]
4:P35247
   Pulmonary surfactant-associated protein D precursor (SP-D) (PSP-D)
   gi|464486|sp|P35247|PSPD_HUMAN[464486]
5:P12842
   Pulmonary surfactant-associated protein A precursor (SP-A) (PSP-A) (PSAP)
   gi|131413|sp|P12842|PSPA_RABIT[131413]
6: NP_033186
   surfactant associated protein D [Mus musculus]
   gi|6677921|ref|NP_033186.1|[6677921]
7: 1 B08C
   Chain C, Lung Surfactant Protein D (Sp-D) (Fragment)
   gi|6573321|pdb|1B08|C[6573321]
8: 1B08B
   Chain B, Lung Surfactant Protein D (Sp-D) (Fragment)
   gi|6573320|pdb|1B08|B[6573320]
9: 1B08A
   Chain A, Lung Surfactant Protein D (Sp-D) (Fragment)
   gi|6573319|pdb|1B08|A[6573319]
10: NP_060049
   deleted in malignant brain tumors 1 isoform c precursor [Homo sapiens]
   gi|8923740|ref|NP_060049.1|[8923740]
11:NP_015568
   deleted in malignant brain tumors 1 isoform b precursor [Homo sapiens]
   gi|6633801|ref|NP_015568.1|[6633801]
12: NP_004397
   deleted in malignant brain tumors 1 isoform a precursor [Homo sapiens]
   gi|4758170|ref|NP_004397.1|[4758170]
13: LNBOC1
   pulmonary surfactant protein C - bovine
   gi|7428752|pir|LNBOC1[7428752]
14: LNDGC1
   pulmonary surfactant protein C - dog
   gi|7428750|pir||LNDGC1[7428750]
15: JN0450
   conglutinin precursor - bovine
   gi|346501|pir||JN0450[346501]
16:A45225
   pulmonary surfactant protein D precursor - human
   gi|346375|pir∥A45225[346375]
17: LNHUC
   pulmonary surfactant protein C precursor, long splice form - human
   gi|71983|pir||LNHUC[71983]
18: LNDGPS
   pulmonary surfactant protein A precursor - dog
   gi|71970|pir||LNDGPS[71970]
19: LNHUPS
   pulmonary surfactant protein A precursor (genomic clone) - human
   gi|71967|pir||LNHUPS[71967]
20: A53570
   collectin-43 - bovine
   gi|1083017|pir||A53570[1083017]
21: S33603
   surfactant protein D - bovine
   gi|423283|pir||S33603[423283]
22: AAF28384
   lung surfactant protein A [Sus scrofa]
   gi|6782434|gb|AAF28384.1|AF133668_1[6782434]
23: AAF22145
   lung surfactant protein D precursor; SPD; SP-D; CP4 [Sus scrofa]
   gi|6760482|gb|AAF22145.2|AF132496_1[6760482]
24: P 15783
   PULMONARY SURFACTANT-ASSOCIATED PROTEIN C (SP-C) (PULMONARY SURFACTANT-ASSOCIATED PROTEOLIPID SPL(VAL))
   gi|131422|sp|P15783|PSPC_BOVIN[131422]
25: P35246
   PULMONARY SURFACTANT-ASSOCIATED PROTEIN D PRECURSOR (SP-D) (PSP-D)
   gi|464485|sp|P35246|PSPD_BOVIN[464485]
26:P42916
   COLLECTIN-43 (CL-43)
   gi|1168967|sp|P42916|CL43_BOVIN[1168967]
27: CAB56155
   DMBT1/8kb.2 protein [Homo sapiens]
   gi|5912464|emb|CAB56155.1|[5912464]
28: AAD49696
   gp-340 variant protein [Homo sapiens]
   gi|5733598|gb|AAD49696.1|AF159456_1[5733598]
29: AAD31380
   surfactant protein D precursor [Mus musculus]
   gi|4877556|gb|AAD31380.1|AF047742_1[4877556]
30:B61249
   pulmonary surfactant protein C - dog
   gi|539712|pir||B61249[539712]
31:S00609
   pulmonary surfactant protein C - bovine
   gi|89749|pir∥S00609[89749]
32: A43628
   pulmonary surfactant protein A - human (fragments)
   gi|280854|pir||A43628[280854]
33: AAB48076
   Surfactant protein B (SP-B) [Oryctolagus cuniculus]
   gi|1850933|gb|AAB48076.1|[1850933]
34: 1901176A
   surfactant protein A
   gi|382753|prf||1901176A[382753]
35: CAA53510
   lung surfactant protein D [Bos taurus]
   gi|415939|emb|CAA53510.1|[415939]
36: CAA53511
   collectin-43 [Bos taurus]
   gi|499385|emb|CAA53511.1|[499385]
37: CAA46152
   lung surfactant protein D [Homo sapiens]
   gi|34767|emb|CAA46152.1|[34767]
38: AAA92788
   lung surfactant protein C [Rattus norvegicus]
   gi|595282|gb|AAA92788.1|[595282]
39: AAA31468
   surfactant protein A [Oryctolagus cuniculus]
   gi|431446|gb|AAA31468.1|[431446]

### Mannose binding lectin

1: Q9NPY3
   Complement component C1q receptor precursor (Complement component 1, q subcomponent, receptor 1) (C1qRp) (C1qR(p)) (C1q/MBL/SPA receptor) (CD93 antigen) (CDw93)
   gi|21759074|sp|Q9NPY3|CD93_HUMAN[21759074]
2: 089103
   Complement component C1q receptor precursor (Complement component 1, q subcomponent, receptor 1) (C1qRp) (C1qR(p)) (C1q/MBL/SPA receptor) (CD93 antigen) (Cell surface antigen AA4) (Lymphocyte antigen 68)
   gi|21541998|sp|089103|CD93_MOUSE[21541998]
3: P09871
   Complement C1s component precursor (C1 esterase)
   gi|115205|sp|P09871|C1S_HUMAN[115205]
4: NP_036204
   complement component 1, q subcomponent, receptor 1; complement component C1q
   receptor [Homo sapiens]
   gi|6912282|ref|NP_036204.1|[6912282]
5: NP_000233
   soluble mannose-binding lectin precursor; mannose-binding lectin; mannose binding protein; Mannose-binding lectin 2, soluble (opsonic defect) [Homo sapiens]
   gi|4557739|ref|NP_000233.1|[4557739]
6: AAM94381
   lectin precursor [Zephyranthes candida]
   gi|22212748|gb|AAM94381.1|AF527385_1[22212748]
7: AAH21762
   mannose binding lectin, liver (A) [Mus musculus]
   gi|18256010|gb|AAH21762.1|[18256010]
8: AAH10760
   Similar to mannose binding lectin, serum (C) [Mus musculus]
   gi|14789670|gb|AAH10760.1|[14789670]
9:P11226.
   Mannose-binding protein C precursor (MBP-C) (MBP1) (Mannan-binding protein) (Mannose-binding lectin)
   gi|126676|sp|P11226|MABC_HUMAN[126676]
10: NP_034897
   mannan-binding lectin serine protease 2 [Mus musculus]
   gi|6754642|ref|NP_034897.1|[6754642]
11: Q9ET61
   Complement component C1q receptor precursor (Complement component 1, q subcomponent, receptor 1) (C1qRp) (C1qR(p)) (C1q/MBL/SPA receptor) (CD93 antigen) (Cell surface antigen AA4)
   gi|21541989|sp|Q9ET61|CD93_RAT[21541989]
12: NP_006601
   mannan-binding lectin serine protease 2, isoform 1 precursor; MBL-associated plasma protein of 19 kD; small MBL-associated protein [Homo sapiens]
   gi|21264363|ref|NP_006601.2|[21264363]
13: NP_631947
   mannan-binding lectin serine protease 2, isoform 2 precursor; MBL-associated plasma protein of 19 kD; small MBL-associated protein [Homo sapiens]
   gi|21264361|ref|NP_631947.1|[21264361]
14: NP_624302
   mannan-binding lectin serine protease 1, isoform 2, precursor; protease, serine, 5 (mannose-binding protein-associated); manan-binding lectin serine protease-1; Ra-reactive factor serine protease p100 [Homo sapiens]
   gi|21264359|ref|NP_624302.1|[21264359]
15: NP_001870
   mannan-binding lectin serine protease 1, isoform 1, precursor; protease, serine, 5 (mannose-binding protein-associated); manan-binding lectin serine protease-1; Ra-reactive factor serine protease p100 [Homo sapiens]
   gi|21264357|ref|NP_001870.3|[21264357]
16: XP_122683
   similar to mannose binding lectin, liver (A) [Mus musculus]
   gi|20872845|ref|XP_122683.1|[20872845]
17: AAM21196
   C-type mannose-binding lectin [Oncorhynchus mykiss]
   gi|20385163|gb|AAM21196.1|AF363271_1[20385163]
18: AAD45377
   mannose-binding lectin [Sus scrofa]
   gi|5566370|gb|AAD45377.1|AF164576_1[5566370]
19: NP_034905
   mannose binding lectin, liver (A) [Mus musculus]
   gi|6754654|ref|NP_034905.1|[6754654]
20: NP_034906
   mannose binding lectin, serum (C) [Mus musculus]
   gi|6754656|ref|NP_034906.1|[6754656]
21: AAL14428
   dendritic cell-specific ICAM-3 grabbing nonintegrin [Macaca nemestrina]
   gi|16118455|gb|AAL14428.1|AF343727_1[16118455]
22: AAF83470
   mannose binding-like lectin precursor [Carassius auratus]
   gi|7542474|gb|AAF63470.1|AF227739_1[7542474]
23: AAF63469
   mannose binding-like lectin precursor [Danio rerio]
   gi|7542472|gb|AAF63469.1|AF227738_1[7542472]
24: AAF63468
   mannose binding-like lectin precursor [Cyprinus carpio]
   gi|7542470|gb|AAF63468.1|AF227737_1[7542470]
25:AAF21018
   mannose-binding lectin 2 [Sus scrofa]
   gi|6644342|gb|AAF21018.1|AF208528_1[6644342]
26: AAK30298
   mannose-binding lectin precursor protein [Gallus gallus]
   gi|13561409|gb|AAK30298.1|[13561409]
27: LNMSMC
   mannose-binding lectin C precursor - mouse
   gi|7428747|pir||LNMSMC[7428747]
28: LNMSMA
   mannose-binding lectin A precursor - mouse
   gi|625320|pir||LNMSMA[625320]
29: LNRTMA
   mannose-binding lectin A precursor - rat
   gi|71975|pir||LNRTMA[71975]
30: LNRTMC
   mannose-binding lectin C precursor - rat
   gi|71974|pir∥LNRTMC[71974]
31: LNHUMC
   mannose-binding lectin precursor - human
   gi|71973|pir||LNHUMC[71973]
32: BAA86864
   complement C1s [Homo sapiens]
   gi|6407558|dbj|BAA86864.1|[6407558]
33:P49329
   MANNOSE-SPECIFIC LECTIN (AGGLUTININ)
   gi|1346426|sp|P49329|LEC_ALOAR[1346426]
34: CAB56124
   mannose-binding lectin [Homo sapiens]
   gi|5911809|emb|CAB56124.1|[5911809]
35: CAB56123
   mannose-binding lectin [Homo sapiens]
   gi|5911807|emb|CAB56123.1|[5911807]
36: CAB56122
   mannose-binding lectin [Homo sapiens]
   gi|5911798|emb|CAB56122.1|[5911798]
37: CAB56121
   mannose-binding lectin [Homo sapiens]
   gi|5911796|emb|CAB56121.1|[5911796]
38: CAB56045
   mannose-binding lectin [Homo sapiens]
   gi|5911794|emb|CAB56045.1|[5911794]
39: CAB56120
   mannose-binding lectin [Homo sapiens]
   gi|5911792|emb|CAB56120.1|[5911792]
40: CAB56044
   mannose-binding lectin [Homo sapiens]
   gi|5911790|emb|CAB56044.1|[5911790]
41: AAB53110
   C1qR(p) [Homo sapiens]
   gi|2052498|gb|AAB53110.1|[2052498]

The collectin preferably comprises at least 10, such as at least 12, for example at least 15, such as at least 20, for example at least 25, such as at least 30, for example at least 35, such as at least 40, for example at least 50 consecutive amino acid residues of the collectin or of a variant or a homologue to said collectin. Such a variant or homologue is preferably at least 70%, such as 80%, for example 90%, such as 95% identical to the collectin.

### Ficolins

The ficolin according to the invention may be L-ficolin, H-ficolin or M-ficolin or variants or homologues thereof. In a preferred embodiment the ficolin is L-ficolin.

In a particular preferred embodiment the ficolin has one of the sequences listed below with reference to their database and accession No. For each of the sequences the Cystein rich region and the collagen-like region is described.

NP_003656. ficolin 3 precursor; ficolin (collagen/fibrinogen domain-containing) 3 (Hakata antigen) [Homo sapiens] [gi:4504331]
90..299 /region_name="pfam00147, fibrinogen_C, Fibrinogen beta and gamma chains, C-terminal globular domain"
90..299 /region_name="smart00186, FBG, Fibrinogen-related domains (FReDs); Domain present at the C-termini of fibrinogen beta and gamma chains, and a variety of fibrinogen-related proteins, including tenascin and Drosophila scabrous"

| | |
|---|---|
| 1 | mdllwilpsl wllllggpac lktqehpscp gpreleaskv vllpscpgap gspgekgapg |
| 61 | pqgppgppgk mgpkgepgdp vnllrcqegp rncrellsqg atlsgwyhlc lpegralpvf |
| 121 | cdmdtegggw lvfqrrqdgs vdffrswssy ragfgnqese fwlgnenlhq Itlqgnwelr |
| 181 | veledfngnr tfahyatfrl Igevdhyqla lgkfsegtag dslslhsgrp fttydadhds |
| 241 | snsncavivh gawwyascyr snlngryavs daaahkygid wasgrgvghp yrrvrmmlr |

XP_116792. similar to Ficolin 2 precursor (Collagen/fibrinogen domain-containing protein 2) (Ficolin-B) (Ficolin B) (Serum lectin P35) (EBP-37) (Hucolin) (L-Ficolin) [Homo sapiens] [gi:20477458]
91..168 /region_name="pfam00147, fibrinogen_C, Fibrinogen beta and gamma chains, C-terminal globular domain"
91..168 /region_name="smart00186, FBG, Fibrinogen-related domains (FReDs);
Domain present at the C-termini of fibrinogen beta and gamma chains, and a variety of fibrinogen-related proteins, including tenascin and Drosophila scabrous"

| | |
|---|---|
| 1 | mgpallalsf lwtmaltedt cpamleyval nsepgmaskn psrrhglsll wdqgpgarg |
| 61 | vrtdqgpsga dpgslelhge cpifpseqvi lthhnnypfs tedqdndrda encavhyqga |
| 121 | wwyaschlsh Ingvylggar dsftnginwk sgkgnnysyk vsemkvrpt |

000602. Ficolin 1 precursor (Collagen/fibrinogen domain-containing protein 1) (Ficolin-A) (Ficolin A) (M-Ficolin) [gi:20455484]
1..29 /gene="FCN1" /region_name="Signal" /note="POTENTIAL."
30..326 /gene="FCN1" /region_name="Mature chain" /note="FICOLIN 1."
55..93 /gene="FCN1" /region_name="Domain" /note="COLLAGEN-LIKE."
133 /gene="FCN1" /region_name="Conflict" /note="T -> N (IN REF. 1)."
144..290 /gene="FCN1" /region_name="Domain" /note="FIBRINOGEN C-TERMINAL."
287 /gene="FCN1" /region_name="Conflict" /note="N -> S (IN REF. 1)."
305 /gene="FCN1" /site_type="glycosylation" /note="N-LINKED (GLCNAC...) (POTENTIAL)."

| | |
|---|---|
| 1 | melsgatmar glavllvlfl hiknlpaqaa dtcpevkwg legsdkltil rgcpglpgap |
| 61 | gpkgeagvig ergerglpga pgkagpvgpk gdrgekgmrg ekgdagqsqs catgprnckd |
| 121 | Ildrgyflsg whtiylpdcr pltvlcdmdt dgggwtvfqr rmdgsvdfyr dwaaykqgfg |
| 181 | sqlgefwlgn dnihaltaqg sselrvdlvd fegnhqfaky ksfkvadeae kyklvlgafv |
| 241 | ggsagnsltg hnnnffstkd qdndvsssnc aekfqgawwy adchasnlng lylmgphesy |
| 301 | anginwsaak gykysykvse mkvrpa // |

075636. Ficolin 3 precursor (Collagen/fibrinogen domain-containing protein 3) (Collagen/fibrinogen domain-containing lectin 3 P35) (Hakata antigen) [gi:13124185]
1..21 /gene="FCN3" /region_name="Signal" /note="POTENTIAL."
22..299 /gene="FCN3" /region_name="Mature chain" /note="FICOLIN 3."
48..80 /gene="FCN3" /region_name="Domain" /note="COLLAGEN-LIKE."
50 /gene="FCN3" /site_type="hydroxylation"
53 /gene="FCN3" /site_type="hydroxylation"
59 /gene="FCN3" /site_type="hydroxylation"
65 /gene="FCN3" /site_type="hydroxylation"
68 /gene="FCN3" /site_type="hydroxylation"
77 /gene="FCN3" /site_type="hydroxylation"
119..265 /gene="FCN3" /region_name="Domain" /note="FIBRINOGEN C-TERMINAL."
189 /gene="FCN3" /site_type="glycosylation" /note="N-LINKED (GLCNAC...) (POTENTIAL)."

| | |
|---|---|
| 1 | mdllwilpsl wllllggpac lktqehpscp gpreleaskv vllpscpgap gspgekgapg |
| 61 | pqgppgppgk mgpkgepgdp vnllrcqegp rncrellsqg atlsgwyhlc lpegralpvf |
| 121 | cdmdtegggw lvfqrrqdgs vdffrswssy ragfgnqese fwlgnenlhq ltlqgnwelr |
| 181 | veledfngnr tfahyatfrl Igevdhyqla lgkfsegtag dslslhsgrp fttydadhds |
| 241 | snsncavivh gawwyascyr snlngryavs daaahkygid wasgrgvghp yrrvrmmlr |

XP_130120. similar to Ficolin 2 precursor (Collagen/fibrinogen domain-containing protein 2) (Ficolin-B) (Ficolin B) (Serum lectin P35) (EBP-37) (Hucolin) [Mus musculus] [gi:20823464]
59..95 /region_name="Collagen triple helix repeat (20 copies)" /note="Collagen" /db_xref="CDD:pfam01391"
59..89 /region_name='Collagen triple helix repeat (20 copies)" /note="Collagen" /db_xref="CDD:pfam01391"
60..95 /region_name="Collagen triple helix repeat (20 copies)" /note="Collagen" /db_xref="CDD:pfam01391"
60..95 /region_name="Collagen triple helix repeat (20 copies)" /note="Collagen" /db_xref="CDD:pfam01391"
60..95 /region_name="Collagen triple helix repeat (20 copies)" /note="Collagen" /db_xref="CDD:pfam01391"
60..95 /region_name="Collagen triple helix repeat (20 copies)" /note="Collagen" /db_xref="CDD:pfam01391"
60..95 /region_name="Collagen triple helix repeat (20 copies)" /note="Collagen" /db_xref="CDD:pfam01391"
61..95 /region_name="Collagen triple helix repeat (20 copies)" /note="Collagen" /db_xref="CDD:pfam01391"
61..95 /region_name="Collagen triple helix repeat (20 copies)" /note="Collagen" /db_xref="CDD:pfam01391"
61..95 /region_name="Collagen triple helix repeat (20 copies)" /note="Collagen" /db_xref="CDD:pfam01391" 103..312 /region_name="Fibrinogen beta and gamma chains, C-terminal globular domain" /note="fibrinogen_C" /db_xref="CDD:pfam00147"
103..312 /region_name="Fibrinogen-related domains (FReDs)" /note="FBG" /db_xref="CDD:smart00186"

| | |
|---|---|
| 1 | malgsaalfv ltltvhaagt cpelkvldle gykqltilqg cpglpgaagp kgeagakgdr |
| 61 | gesglpgipg kegptgpkgn qgekgirgek gdsgpsqsca tgprtckell tqghfitgwy |
| 121 | tiylpdcrpl tvlcdmdtdg ggwtvfqrrl dgsvdffrdw tsykrgfgsq lgefwlgndn |
| 181 | ihaittqgts elrvdlsdfe gkhdfakyss fqiqgeaeky klilgnflgg gagdsltphn |
| 241 | nrlfstkdqd ndgstsscam gyhgawwysq chtsningly lrgphksyan gvnwkswrgy |
| 301 | nysckvsemk vrfi |

NP_056654. ficolin 2 isoform d precursor; ficolin (collagen/fibrinogen domain-containing lectin) 2 (hucolin); ficolin (collagen/fibrinogen domain-containing lectin) 2; hucolin [Homo sapiens] [gi:8051590]
39..95 /region_name="collagen-like domain"

| | |
|---|---|
| 1 | meldravgvl gaatlllsfl gmawalqaad tcpevkmvgl egsdkltilr gcpglpgapg |
| 61 | dkgeagtngk rgergppgpp gkagppgpng apgepqpclt gd |

NP_056653. ficolin 2 isoform c precursor; ficolin (collagen/fibrinogen domain-containing lectin) 2 (hucolin); ficolin (collagen/fibrinogen domain-containing lectin) 2; hucolin [Homo sapiens] [gi:8051588]
39..95 /region_name="collagen-like domain"
102..143 /region_name="Fibrinogen beta and gamma chains, C-terminal globular domain" /note="fibrinogen_C" /db_xref="CDD:pfam00147"
102..143 /region_name="Fibrinogen-related domains (FReDs)" /note="FBG" /db_xref="CDD:smart00186"

| | |
|---|---|
| 1 | meldravgvl gaatlllsfl gmawalqaad tcpevkmvgl egsdkltilr gcpglpgapg |
| 61 | dkgeagtngk rgergppgpp gkagppgpng apgepqpclt gprtckdlld rghflsgwht |
| 121 | iylpdcrplt vlcdmdtdgg gwtvsvglgr ggqpgspggq aahlvgehtl efsillvgds |
| 181 | qr |

NP_056652. ficolin 2 isoform b precursor; ficolin (collagen/fibrinogen domain-containing lectin) 2 (hucolin); ficolin (collagen/fibrinogen domain-containing lectin) 2; hucolin [Homo sapiens] [gi:8051586]
sig peptide 1..25
mat peptide 26..275
60..275 /region_name="FBG domain" /note="fibrinogen beta/gamma homology"
64..275 /region_name="Fibrinogen-related domains (FReDs)" /note="FBG" /db_xref="CDD:smart00186"
64..274 /region_name="Fibrinogen beta and gamma chains, C-terminal globular domain" /note="fibrinogen_C" /db_xref="CDD:pfam00147"

| | |
|---|---|
| 1 | meldravgvl gaafilllsfl gmawalqaad tcpgergppg ppgkagppgp ngapgepqpc |
| 61 | ltgprtckdl ldrghfisgw htiylpdcrp ltvlcdmdtd gggwtvfqrr vdgsvdfyrd |
| 121 | watykqgfgs rlgefwlgnd nihaltaqgt selrvdlvdf ednyqfakyr sfkvadeaek |
| 181 | ynlvlgafve gsagdsltfh nnqsfstkdq dndlntgnca vmfqgawwyk nchvsnlngr |
| 241 | ylrgthgsfa nginwksgkg ynysykvsem kvrpa |

NP_001994. ficolin 1 precursor; ficolin (collagen/fibrinogen domain-containing) 1 [Homo sapiens] [gi:8051584]
sig peptide 1..27
mat peptide 28..326 40..108 /region_name="collagen-like domain"
50..105 /region_name="Collagen triple helix repeat (20 copies)" /note="Collagen" /db_xref="CDD:pfam01391"
51..107./region_name="Collagen triple helix repeat (20 copies)" /note="Collagen" /db_xref="CDD:pfam01391"
52..106 /region_name="Collagen triple helix repeat (20 copies)" /note="Collagen" /db_xref="CDD:pfam01391"
115..326 /region_name="FBG domain" /note="fibrinogen beta/gamma homology"
115..326 /region_name="Fibrinogen-related domains (FReDs)" /note="FBG"
/db_xref="CDD:smart00186"
115..325/region_name="Fibrinogen beta and gamma chains, C-terminal globular domain" /note="fibrinogen_C" /db_xref="CDD:pfam00147" variation 315
/db_xref="dbSNP:1128428" variation 316 /db_xref="dbSNP:1128429" variation 317 /db_xref="dbSNP:1128430"

| | |
|---|---|
| 1 | melsgatmar glavllvlfl hiknlpaqaa dtcpevkwg legsdkltil rgcpglpgap |
| 61 | gpkgeagvig ergerglpga pgkagpvgpk gdrgekgmrg ekgdagqsqs catgprnckd |
| 121 | lldrgyflsg whtiylpdcr pltvlcdmdt dgggwtvfqr rmdgsvdfyr dwaaykqgfg |
| 181 | sqlgefwlgn dnihaltaqg sselrvdlvd fegnhqfaky ksfkvadeae kyklvlgafv |
| 241 | ggsagnsltg hnnnffstkd qdndvsssnc aekfqgawwy adchasnlng lylmgphesy |
| 301 | anginwsaak gykysykvse mkvrpa |

NP_004099. ficolin 2 isoform a precursor; ficolin (collagen/fibrinogen domain-containing lectin) 2 (hucolin); ficolin (collagen/fibrinogen domain-containing lectin) 2; hucolin [Homo sapiens] [gi:4758348]
sig peptide 1..25
mat peptide 26..313
39..95 /region_name="collagen-like domain"
98..313 /region_name="FBG domain" /note="fibrinogen beta/gamma homology"
102..313 /region_name="Fibrinogen-related domains (FReDs)" /note="FBG" /db_xref="CDD:smart00186"
102..312 /region_name="Fibrinogen beta and gamma chains, C-terminal globular domain" /note="fibrinogen_C" /db_xref="CDD:pfam00147

| | |
|---|---|
| 1 | meldravgvl gaatlllsfl gmawalqaad tcpevkmvgl egsdkltilr gcpglpgapg |
| 61 | dkgeagtngk rgergppgpp gkagppgpng apgepqpclt gprtckdlld rghflsgwht |
| 121 | iylpdcrplt vlcdmdtdgg gwtvfqrrvd gsvdfyrdwa tykqgfgsrl gefwlgndni |
| 181 | haltaqgtse Irvdlvdfed nyqfakyrsf kvadeaekyn Ivlgafvegs agdsltfhnn |
| 241 | qsfstkdqdn dlntgncavm fqgawwyknc hvsnlngryl rgthgsfang inwksgkgyn |
| 301 | ysykvsemkv rpa |

Q9WTS8. Ficolin 1 precursor (Collagen/fibrinogen domain-containing protein 1) (Ficolin-A) (Ficolin A) (M-Ficolin) [gi:13124116]
1..22 /gene="FCN1" /region_name="Signal" /note="POTENTIAL."
23..335 /gene="FCN1" /region_name="Mature chain" /note="FICOLIN 1."
50..88 /gene="FCN1" /region_name="Domain" /note="COLLAGEN-LIKE."
152..298 /gene="FCN1" /region_name="Domain" /note="FIBRINOGEN C-TERMINAL."
271 /gene="FCN1" /site_type="glycosylation" /note="N-LINKED (GLCNAC...) (POTENTIAL)."

| | |
|---|---|
| 1 | mwwpmlwafp vllclcssqa lgqesgacpd vkivglgaqd kvaviqscps fpgppgpkge |
| 61 | pgspagrger glqgspgkmg ppgskgepgt mgppgvkgek gergtasplg qkelgdalcr |
| 121 | rgprsckdll trgifltgwy tiylpdcrpl tvlcdmdvdg ggwtvfqrrv dgsinfyrdw |
| 181 | dsykrgfgnl gtefwlgndy Ihlltangnq elrvdlrefq gqtsfakyss fqvsgeqeky |
| 241 | kltlgqfleg tagdsltkhn nmafsthdqd ndtnggknca alfhgawwyh dchqsnlngr |
| 301 | ylpgshesya dginwlsgrg hrysykvaem kiras |

Q15485. Ficolin 2 precursor (Collagen/fibrinogen domain-containing protein 2) (Ficolin-B) (Ficolin B) (Serum lectin P35) (EBP-37) (Hucolin) (L-Ficolin) [gi:13124203]
1..25 /gene="FCN2" /region_name="Signal" /note="POTENTIAL."
26..313 /gene="FCN2" /region_name="Mature chain" /note="FICOLIN 2."
54..92 /gene="FCN2" /region_name="Domain" /note="COLLAGEN-LIKE."
131..277 /gene="FCN2" /region_name="Domain" /note="FIBRINOGEN C-TERMINAL."
240 /gene="FCN2" /site_type="glycosylation" /note="N-LINKED (GLCNAC...) (POTENTIAL)."
300 /gene="FCN2" /site_type="glycosylation" /note="N-LINKED (GLCNAC...) (POTENTIAL)."

| | |
|---|---|
| 1 | meldravgvl gaatlllsfl gmawalqaad tcpevkmvgl egsdkltilr gcpglpgapg |
| 61 | dkgeagtngk rgergppgpp gkagppgpng apgepqpclt gprtckdlld rghflsgwht |
| 121 | iylpdcrplt vlcdmdtdgg gwtvfqrrvd gsvdfyrdwa tykqgfgsrl gefwlgndni |
| 181 | haltaqgtse Irvdlvdfed nyqfakyrsf kvadeaekyn Ivlgafvegs agdsltfhnn |
| 241 | qsfstkdqdn dlntgncavm fqgawwyknc hvsnlngryl rgthgsfang inwksgkgyn |
| 301 | ysykvsemkv rpa |

070497. Ficolin 2 precursor (Collagen/fibrinogen domain-containing protein 2) (Ficolin-B) (Ficolin B) (Serum lectin P35) (EBP-37) (Hucolin) [gi:13124181]
<1..15 /gene="FCN2" /region_name="Signal" /note="POTENTIAL."
16..>306 /gene="FCN2" /region_name="Mature chain" lnote="FICOLIN 2." 41..79 /gene="FCN2" /region_name="Domain" /note="COLLAGEN-LIKE."
130..276 /gene="FCN2" /region_name="Domain" /note="FIBRINOGEN C-TERMINAL."
299 /gene="FCN2" /site_type="glycosylation" /note="N-LINKED (GLCNAC...) (POTENTIAL)."

| | |
|---|---|
| 1 | Igsaalfvlt Itvhaagtcp elkvldlegy kqltilqgcp glpgaagpkg eagakgdrge |
| 61 | sglpgipgke gptgpkgnqg ekgirgekgd sgpsqscatg prtckelltq ghfltgwyti |
| 121 | ylpdcrpmtv lcdmdtdggg wtvfqrrldg svdffrdwts ykrgfgsqlg efwlgndnih |
| 181 | alttqgtsel rvdlsdfegk hdfakyssfq iqgeaekykl ilgnflggga gdsltphnnr |
| 241 | lfstkdqdnd gstsscamgy hgawwysqch tsnlnglylr gphksyangv nwkswrgyny |
| 301 | sckvse |

070165. Ficolin 1 precursor (Collagen/fibrinogen domain-containing protein 1) (Ficolin-A) (Ficolin A) (M-Ficolin) [gi:13124179]
1..22 /gene="FCN1" /region_name="Signal" /note="POTENTIAL."
23..334 /gene="FCN1" /region_name="Mature chain" /note="FICOLIN 1."
50..88 /gene="FCN1" /region_name="Domain" /note="COLLAGEN-LIKE."
152..298 /gene="FCN1" /region_name="Domain" /note="FIBRINOGEN C-TERMINAL."
261 /gene="FCN1" /site_type="glycosylation" /note="N-LINKED (GLCNAC...) (POTENTIAL)."

| | |
|---|---|
| 1 | mqwptlwafs gllclcpsqa Igqergacpd vkvvglgaqd kvwiqscpg fpgppgpkge |
| 61 | pgspagrger gfqgspgkmg pagskgepgt mgppgvkgek gdtgaapslg ekelgdtlcq |
| 121 | rgprsckdll trgifltgwy tihlpdcrpl tvlcdmdvdg ggwtvfqrrv dgsidffrdw |
| 181 | dsykrgfgnl gtefwlgndy Ihlltangnq elrvdlqdfq gkgsyakyss fqvseeqeky |
| 241 | kltlgqfleg tagdsltkhn nmsftthdqd ndansmncaa lfhgawwyhn chqsnlngry |
| 301 | Isgshesyad ginwgtgqgh hysykvaemk iras |

P57756. Ficolin 2 precursor (Collagen/fibrinogen domain-containing protein 2) (Ficolin-B) (Ficolin B) (Serum lectin P35) (EBP-37) (Hucolin) [gi:13124114]
1..22 /gene="FCN2" /region_name="Signal" /note="POTENTIAL." 23..319 /gene="FCN2" /region_name="Mature chain" /note="FICOLIN 2." 48..86 /gene="FCN2" /region_name="Domain" /note="COLLAGEN-LIKE."
137..283 /gene="FCN2" /region_name="Domain" /note="FIBRINOGEN C-TERMINAL."
306 /gene="FCN2" /site_type="glycosylation" /note="N-LINKED (GLCNAC...) (POTENTIAL)."

| | |
|---|---|
| 1 | mvlgsaalfv Islcvteltl haadtcpevk vldlegsnkl tilqgcpglp galgpkgeag |
| 61 | akgdrgesgl pghpgkagpt gpkgdrgekg vrgekgdtgp sqscatgprt ckelltrgyf |
| 121 | ltgwytiylp dcrpltvlcd mdtdgggwtv fqrridgtvd ffrdwtsykq gfgsqlgefw |
| 181 | Igndnihalt tqgtnelrvd ladfdgnhdf akyssfqiqg eaekyklilg nflgggagds |
| 241 | ltsqnnmlfs tkdqdndqgs sncavryhga wwysdchtsn Inglylrglh ksyangvnwk |
| 301 | swkgynysyk vsemkvrli |

JC5980. ficolin-A precurs - mouse [gi:7513652]
1..21 /region_name="domain" /note="signal sequence"
50..64 /region_name="domain" /note="collagen-like" 68..106 /region_name="domain" /note="collagen-like"
123..334 /region_name="domain" /note="fibrinogen beta/gamma homology #label FBG"

| | |
|---|---|
| 1 | mqwptlwafs gllclcpsqa lgqergacpd vkwglgaqd kwviqscpg fpgppgpkge |
| 61 | pgspagrger gfqgspgkmg pagskgepgt mgppgvkgek gdtgaapslg ekelgdtlcq |
| 121 | rgprsckdll trgifltgwy tihlpdcrpl tvlcdmdvdg ggwtvfqrrv dgsidffrdw |
| 181 | dsykrgfgnl gtefwlgndy Ihlltangnq elrvdlqdfq gkgsyakyss fqvseeqeky |
| 241 | kltlgqfleg tagdsltkhn nmsftthdqd ndansmncaa lfhgawwyhn chqsnlngry |
| 301 | lsgshesyad ginwgtgqgh hysykvaemk iras |

S61517. ficolin-1 precurs- human [gi:2135116]
1..326 /note="36K HLA-cross-reactive plasma protein; hucolin, 35K"
1..22 /region_name="domain" /note="signal sequence"
52..108 /region_name="region" /note="collagen-like"
115..326 /region_name="domain" /note="fibrinogen beta/gamma homology #label FBG"
305 /site_type="binding" /note="carbohydrate (Asn) (covalent)"

| | |
|---|---|
| 1 | melsgatmar glavllvlfl hiknlpaqaa dtcpevkvvg legsdkltil rgcpglpgap |
| 61 | gpkgeagvig ergerglpga pgkagpvgpk gdrgekgmrg ekgdagqsqs catgprnckd |
| 121 | Ildrgyflsg whniylpdcr pltvlcdmdt dgggwtvfqr rmdgsvdfyr dwaaykqgfg |
| 181 | sqlgefwlgn dnihaltaqg sselrvdlvd fegnhqfaky ksfkvadeae kyklvlgafv |
| 241 | ggsagnsltg hnnnffstkd qdndvsssnc aekfqgawwy adchasslng lylmgphesy |
| 301 | anginwsaak gykysykvse mkvrpa |

A47172. transforming growth factor-beta 1-binding protein homolog ficolin-alpha - pig [gi:423206]
112..323 /region_name="domain" /note="fibrinogen beta/gamma homology #label FBG"

| | |
|---|---|
| 1 | mdtrgvaaam rplvllvafl ctaapaldtc pevkwgleg sdklsilrgc pglpgaagpk |
| 61 | geagasgpkg gqgppgapge pgppgpkgdr gekgepgpkg esweteqclt gprtckellt |
| 121 | rghilsgwht iylpdcqplt vlcdmdtdgg gwtvfqrrsd gsvdfyrdwa aykrgfgsql |
| 181 | gefwlgndhi haltaqgtne lrvdlvdfeg nhqfakyrsf qvadeaekym Ivlgafvegn |
| 241 | agdsltshnn slfttkdqdn dqyasncavl yqgawwynsc hvsnlngryl ggshgsfang |
| 301 | vnwssgkgyn ysykvsemkf rat |

JC4942. ficolin-1 precursor - human [gi:2135117]
1..22 /region_name="domain" /note="signal sequence"
45..101 /region_name="region" /note="collagen-like"
108..319 /region_name="domain" /note="fibrinogen beta/gamma homology #label FBG"
111..315 /region_name="region" /note="fibrinogen-like"
298 /site_type="binding" /note="carbohydrate (Asn) (covalent)"

| | |
|---|---|
| 1 | marglavllv Iflhiknlpa qaadtcpevk wglegsdkl tilrgcpglp gapgpkgeag |
| 61 | vigergergl pgapgkagpv gpkgdrgekg mrgekgdagq sqscatgprn ckdlldrgyf |
| 121 | Isgwhtiylp dcrpltvlcd mdtdgggwtv fqrrmdgsvd fyrdwaaykq gfgsqlgefw |
| 181 | Igndnihalt aqgsselrvd lvdfegnhqf akyksfkvad eaekyklvlg afvggsagns |
| 241 | Itghnnnffs tkdqdndvss sncaekfqga wwyadchasn Inglylmgph esyanginws |
| 301 | aakgykysyk vsemkvrpa |

AAF44911. symbol=BG:DS00929...[gi:7287873]

| | |
|---|---|
| 1 | mkscffvlfl wtllfevgqs sphtcpsgsp ngihqlmlpe eepfqvtqck ttardwiviq |
| 61 | rrldgsvnfn qswfsykdgf gdpngeffig lqklylmtre qphelfiqlk hgpgatvyah |
| 121 | fddfqvdset elyklervgk ysgtagdslr yhinkrfstf drdndesskn caaehgggww |
| 181 | fhsclsr |

The ficolin preferably comprises at least 10, such as at least 12, for example at least 15, such as at least 20, for example at least 25, such as at least 30, for example at least 35, such as at least 40, for example at least 50 consecutive amino acid residues of the ficolins identified above or of a variant or a homologue to said ficolin. Such a variant or homologue is preferably at least 70%, such as 80%, for example 90%, such as 95% identical to the complement activating protein.

In the following the invention is described in relation to MBL as an example:

SARS may be prevented when administering MBL to these individuals having an MBL level in excess of 10 ng/ml serum. Also, individuals having an MBL level in excess of 50 ng/ml serum may be in need of treatment, such as individuals having an MBL level in excess of 100 ng/ml serum, and individuals having an MBL level in excess of 150 ng/ml serum.

Also the MBL treatment of SARS may be conducted by administering MBL to these individuals in combination with relevant antibiotics, anti-viral agents or anti-fungal agents.

In particular, individuals at risk of acquiring SARS will benefit from being prophylactically treated with MBL.

Generally all individuals exposed to SARS patients should be treated with MBL independent on their specific MBL level. The reason behind this is that SARS may lead to MBL depletion, and therefore an MBL "booster", increasing the MBL level initially will reduce the risk of MBL depletion to a level below a deficiency level, and the immune defence of these patients can be reinforced by administration of recombinant or natural plasma-derived MBL. In particular SARS may be prevented when administering MBL to individuals having an MBL level in excess of 10 ng/ml serum. Also, individuals having an MBL level in excess of 50 ng/ml serum may be in need of treatment, such as individuals having an MBL level in excess of 100 ng/ml serum, and individuals having an MBL level in excess of 150 ng/ml serum.

The present inventors have also shown herein that in particular individuals having an MBL level below 500 ng/ml serum will benefit from the MBL treatment. Consequently, in particular individuals having an MBL level below 400 ng/ml will benefit, such as individuals having an MBL level below 300 ng/ml, such as individuals having an MBL level below 250 ng/ml, such as individuals having an MBL level below 200 ng/ml.

Thus, the present invention provides the use of MBL for manufacturing of a medicament for treatment of individuals having an MBL level in serum in the range of 10-500 ng/ml, such as in the range of 50-500 ng/ml for treating and/or preventing SARS.

One group of individuals being in need of MBL treatment in order to prevent and/or treat SARS are individuals having a low level of functional MBL, independent on the level of MBL as such. This is due to the fact that for some mutations of the MBL it has been found that although MBL subunits and oligomers thereof are expressed in serum the functionality thereof are low. The functionality or functional activity of MBL may be estimated by its capacity to form an MBL/MASP complex leading to activation of the complement system. When C4 is cleaved by MBL/MASP an active thiol-ester is exposed and C4 becomes covalently attached to nearby nucleophilic groups. A substantial part of the C4b will thus become attached to the coated plastic well and may be detected by anti-C4 antibody.

A quantitative TRIFMA for MBL functional activity is constructed by 1) coating microtitre wells with 1 mg mannan in 100 ml buffer; 2) blocking with Tween-20; 3) applying test samples, e.g. diluted MBL preparations 4) applying MBL deficient serum (this leads to the formation of the MBL/MASP complex); alternatively the MBL and the MBL deficient serum may be mixed before application with the microtitre wells; 5) applying purified complement factor C4 at 5 mg/ml; 6) incubating for one hour at 37°C; 7) applying Eu-labelled anti-C4 antibody; 8) applying enhancement solution; and 9) reading the Eu by time resolved fluorometry. Between each step the plate is incubated at room temperature and washed, except between step 8 and 9.

Estimation by ELISA may be carried out similarly, e.g. by applying biotin-labelled anti-C4 in step 7; 8) apply alkaline phosphatase-labelled avidin; 9) apply substrate; and 10) read the colour intensity.

The functionality may be expressed as the specific activity of MBL, such as 1 unit of MBL activity per ng MBL. A non-functional MBL may be defined as MBL having a specific activity less than 50 % of plasma MBL specific activity, such as less than 25 % of plasma MBL specific activity, wherein the plasma MBL is purified from an individual not suffering from any MBL mutations. In particular the reference plasma MBL is plasma pool LJ 6.57 28/04/97.

Thus, the present invention also relates to the prevention and/or treatment of SARS in individuals having a mutation in their MBL gene leading to a reduced expression of MBL and/or expression of non-functional MBL.

In particular such mutations in the MBL gene can lead to a change of aminoacid number 52 (numbering including the leader peptide of MBL) from arginine to cysteine, aminoacid number 54 from glycine to aspartic acid or amino acid number 75 from glycine to glutamic acid.

Also mutations in the promoter region of the MBL gene can lead to lowered levels of MBL. In particular mutations at position -221 have an influence onthe expression of MBL.

The MBL sequence may be found in swiss.prot under accession No: 11226

The MBL composition used to manufacture an MBL medicament may be produced from any MBL source available. The MBL source may be natural MBL, whereby the MBLs are produced in a native host organism, meaning that MBL is produced by a cell normally expressing MBL. One usual method of producing an MBL composition is by extraction of MBL from human body liquids, such as serum or plasma, but MBL may also be harvested from cultures of hepatocytes.

In another aspect the MBL oligomers are produced by a host organism not natively expressing an MBL polypeptide, such as by recombinant technology.

In a first embodiment the MBL source may be serum, from which an MBL composition is obtained by purification from serum, plasma, milk product, colostrum or the like by a suitable purification method, such as affinity chromatography using carbohydrate-derivatised matrices, such as mannose or mannan coupled matrices. Such a method is discussed in WO99/64453, wherein the purification process is followed by a virus-removal step in order to remove infectious agents from the MBL source, since one of the major problems with proteins purified from body liquids is the risk of introducing infectious agents in combination with the desired protein. WO99/64453 is hereby incorporated by reference.

The MBL composition used to manufacture an MBL medicament preferably comprises MBL oligomers having a size distribution substantially identical to the size distribution of MBL in serum, such as a size distribution profile at least 50 % identical to the size distribution profile of MBL in serum. By identical is meant that at least 50 % of the oligomers has an apparent molecular weight higher than 200 kDa, when analysed by SDS-PAGE and/or Western blot.

In a more preferred embodiment the size distribution profile is at least 75 % identical to the size distribution profile of MBL in serum, such as at least 90 % identical to the size distribution profile of MBL in serum, and more preferred at least 95 % identical to the size distribution profile of MBL in serum.

When purifying from an MBL source initially having another size distribution profile it is preferred that the affinity chromatography used to purify from the MBL source favours purification of oligomers having an apparent molecular weight higher than 200 kDa. This is obtained by using a carbohydrate-derivatized matrix having substantially no affinity to subunits and/or dimers of MBL. Preferably the carbohydrate-derivatized matrix has affinity for substantially only tetrameric, pentameric and/or hexameric recombinant MBLs.

The matrix may be derivatized with any carbohydrate or carbohydrate mixture whereto MBL binds and for which binding of the higher oligomers of MBL are favoured. The carbohydrate-derivatized matrix is preferably a hexose-derivatized matrix, such as a mannose- or a N-acetyl-glucosamin derivatized matrix, such as most preferably a mannose-derivatized matrix.

The selectivity of the carbohydrate-derivatized matrix is obtained by securing that the matrix as such, i.e the un-derivatized matrix has substantially no affinity to MBL polypeptides, in particular no affinity to MBL trimers or smaller oligomers. This may be ensured when the matrix as such is carbohydrate-free. In particular the matrix should not contain any Sepharose or the like. It is preferred that the matrix consists of a non-carbohydrate containing polymer material, such as Fractogel®TSK beads

The matrix may be in any form suitable for the chromatography, mostly in the form of beads, such as plastic beads.

After application of the MBL source the column is washed, preferably by using non-denaturing buffers, having a composition, pH and ionic strength resulting in elimination of proteins, without eluting the higher oligomers of MBL. Such as buffer may be TBS. Elution of MBL is performed with a selective desorbing agent, capable of efficient elution of highed oligomers of MBL, such as TBS comprising a desorbing agent, such as EDTA (for example 5 mM EDTA) or mannose (for example 50 mM mannose), and MBL oligomers are collected. Such a purification method is described in co-pending International patent application No. WO 00/70043.

In a preferred aspect a clinical grade MBL composition is obtained by using an MBL source produced by recombinant technology, wherein the MBL source is the culture media from culturing of MBL producing cells.

Thus, the present invention encompasses MBL produced by a process of producing a recombinant mannan binding lectin (MBL), comprising the steps of:
- preparing a gene expression construct comprising a DNA sequence encoding a MBL polypeptide or a functional equivalent thereof,
- transforming a host cell culture with the construct,
- cultivating the host cell culture, thereby obtaining expression and secretion of the polypeptide into the culture medium, followed by
- obtaining a culture medium comprising human recombinant MBLs.

The culture medium comprising the human recombinant MBL polypeptides may then be processed as described above for purification of MBL.

The MBL polypeptide is preferably a mammalian MBL polypeptide, such as more preferably a human MBL polypeptide. The gene expression construct may be produced by conventional methods known to the skilled person, such as described in US patent No. 5,270,199.

In another embodiment the gene expression construct is prepared as described in WO 00/70043.

The expression is preferably carried out in e.g. mammalian cells, the preparation according to the invention results from the use of an expression vector comprising intron sequence(s) from an MBL gene and at least one exon sequence. Regarding the transgenic animals as expression system this term is in this context animals which have been genetically modified to contain and express the human MBL gene or fragments or mimics hereof.

In addition to the purification method it is preferred that the gene expression construct and the host cell also favours production of higher oligomers, which has been found to be possible by using a gene expression construct comprising at least one intron sequence from the human MBL gene or a functional equivalent thereof. malian cells and cells from insects.

Consequently, the MBL composition may be used for preventing and/or treating SARS in an individual wherein the microbial species is a fungus, a yeast, a protozoa, a parasite and/or a bacteria.

The medicament may be produced by using the eluant obtained from the affinity chromatography as such. It is however preferred that the eluant is subjected to further purification steps before being used.

In addition to the MBL oligomers, the medicament may comprise a pharmaceutically acceptable carrier substance and/or vehicles. In particular, a stabilising agent may be added to stabilise the MBL proteins. The stabilising agent may be a sugar alcohol, saccharides, proteins and/or amino acids. Examples of stabilising agents may be maltose or albumin.

Other conventional additives may be added to the medicament depending on administration form for example. In one embodiment the medicament is in a form suitable for injections. Conventional carrier substances, such as isotonic saline, may be used.

In another embodiment the medicament is in a form suitable for pulmonal administration, such as in the form of a powder for inhalation or creme or fluid for topical application.

The route of administration may be any suitable route, such as intravenously, intra-musculary, subcutanously or intradermally. Also, pulmonal or topical administration is envisaged by the present invention.

Normally from 1-100 mg is administered per dosage, such as from 2-10 mg, mostly from 5-10 mg per dosage depending on the individual to be treated, for example about 0.1 mg/kg body weight is administered.

The use of an MBL composition for the manufacture of a medicament may also further comprise the manufacture of another medicament, such as an anti-fungal, anti-yeast, anti-bacterial and/or anti-viral medicament for obtaining a kit-of-parts.

The anti-viral medicament may be a medicament capable of virus attenuation and/or elimination.

The invention also provides an aspect of using a measurement of the MBL level as a prognostic marker for the risk of the individual of acquiring SARS and thereby an indicative of the need for treatment. In particular an MBL level below 500 ng/ml is a prognostic marker indicative for treatment with MBL.

Thus, also disclosed herein is a method of using an MBL composition for preventing and/or treating SARS in an individual, the method comprising the steps of:
i) determining serum levels of MBL in an individual,
ii) estimating the probability of the occurrence of a significant clinical SARS in the individual, and optionally,
administering an MBL composition to the individual.

The MBL level is measured in serum or plasma, and may be determined by time resolved immunofluorescent assay (TRIFMA), ELISA, RIA or nephelometry.

Also the MBL levels may be inferred from analysis of genotypes of the MBL genes as discussed above in relation to mutations of MBL leading to a decreased MBL level.

The invention is illustrated in the following examples.

### Example 1

### MBL serum levels in patients suffering from SARS

Patients are selected among individuals presenting clinically significant SARS as defined above. Patients are identified by retrospective computer search of the patient database.

Before entering treatment blood is drawn into evacuated glass tubes containing EDTA (final concentration about 10 mM). The plasma is aliquoted and kept at -80°C until assay. Plasma samples are similarly obtained from healthy blood donors. The patients are free of infections at the time of blood sampling.

The concentration of MBL is determined by a time resolved immunofluorescent assay (TRIFMA). Microtitre wells (fluoroNunc, Nunc, Kamstrup, Denmark) are coated with antibody by incubation overnight at room temperature with 500 ng anti-human MBL antibody (Mab 131-1, Statens Serum Institut, Copenhagen, Denmark) in 100 µl PBS (0.14 M NaCl, 10 mM phosphate, pH 7.4). After wash with Tween-containing buffer (TBS, 0.14 M NaCl, 10 mM Tris/HCl, 7.5 mM NaN₃, pH 7.4 with 0.05% Tween 20) test samples (plasma 1/20) and calibrator dilutions are added in TBS/Tween with extra NaCl to 0.5 M and 10 mM EDTA.

After overnight incubation at 4°C and wash, the developing europium-labelled antibody (12.5 ng Mab 131-1 labelled with the Eu-containing chelate, isothiocyanatobenzoyl-diethylene-triamine-tetra acetic acid, according to the manufacturer, Wallac, Turku, Finland) is added in TBS/Tween with 25 µM EDTA.

Following incubation for 2 h and wash, fluorescence enhancement solution is added (Wallac) and the plates are read on a time resolved fluorometre (Delfia 1232, Wallac). The calibration curve is made using dilutions of one plasma, which is kept aliquoted at -80°C.

Based on the above outlined method, the MBL serum level of patients with SARS as compared to non-SARS patients is compared.

### Example 2

### Effect of MBL on SARS-related coronavirus (SARS-CoV) infectivity

In the present Example, the following materials were used:
HBSS = Hanks balanced salt solution
PBS = phosphate buffered saline.

### Virus preparation and Cells

Prototype SARS-CoV HKU39849 were grown on the fetal rhesus kidney cells (FRhK-4) (ATCC). The cells were grown in MEM medium with 10% fetal calf serum. Virus titers (TCID₅₀) were determined by titration of a 10-fold dilution series on FRhK-4 cells.

### Assessment of binding of MBL to SARS-CoV strains

Recombinat human MBL (Natimmune A/S) was used.

Binding of MBL to SARS-CoV strains was tested with an ELISA in which suspensions of virus (10³ to 10⁵ TCID₅₀) diluted in PBS were incubated overnight at 4 C on 96-well plates, followed by washing and incubation with MBL.

Before the addition of MBL, plates were blocked with BSA and gelatin in PBS and washed with HBSS+0.05% Tween20.

MBL (0 to 10 µg/mL) diluted in Ca²⁺ containing HBSS were then added and allowed to incubate with virus for 2 hours, followed by the washing off of unbound lectin (three times in HBSS). Control experiments were done by addition of EDTA or mannan to some of the MBL containing wells.

The presence of bound MBL was detected with biotinylated monoclonal anti-MBL (HYB131-01, Antibody shop) diluted in HBSS/ Ca²⁺. Biotin was detected with streptavidin conjugated to horseradish peroxidase and tetramethylbenzidine ('TMB') substrate. The reaction was stopped with 0.5 M H₂SO₄. Absorbance was measured with an ELISA reader. Each individual data point was performed in triplicate. There was minimal background binding of the MBL to wells not containing virus.

### The experiments demonstrated specific binding of MBL to the SARS-CoV.

### Assay of SARS-CoV infectivity

The fetal rhesus kidney cell line (FRhK-4) monolayers were prepared in 96-well plates and grown to confluence. These layers were then infected with SARS-CoV (100 TCID50) previously treated with MBL (0 to 10 µg/mL) for 2 hours at 37 °C. After 2-3 days the monolayers were examined for cythophatic effects (CPE) under light microscope.

*The experiments demonstrated a MBL concentration dependent inhibition of infectivity to the SARS-CoV*.

### References

1. Weis WI, Taylor ME and Drickamer K (1998) The C-type lectin superfamily in the immune system. *Immunological Reviews* 163: 19-34
2. Holmskov, U., Malhotra, R., Sim, R.B., and Jensenius, J.C. (1994) Collectins: collagenous C-type lectins of the innate immune defense system. *Immunol.Today* 15:67-74.
3. Turner, M.W. (1996) Mannose-binding lectin: the pluripotent molecule of the innate immune system. *Immunol.Today* 17:532-540.
4. Janeway CA, Travers P, Walport M and Capra JD (1999) Immunobiology, the immune system in health and disease, Fourth Edition, Churchill Livingstone)
5. Matsushita, M. and Fujita, T (1992). Activation of the classical complement pathway by mannose-binding protein in association with a novel C1s-like serine protease. *J.Exp.Med.* 176:1497-1502.
6. Thiel S, Vorup-Jensen T, Stover CM, Schwaeble W, Laursen SB, Poulsen K, Willis AC, Eggleton P, Hansen S, Holmskov U, Reid KB and Jensenius JC (1997) A second serine protease associated with mannan-binding lectin that activates complement. *Nature,* 386(6624): 506-510
7. Stover CM, Thiel S, Thelen M, Lynch NJ, Vorup-Jensen T, Jensenius JC and Schwaeble WJ (1999) Two constituents of the initiation complex of the mannan-binding lectin activation pathway of complement are encoded by a single structure gene. *J Immunol* 162: 3481-3490
8. Thiel S, Holmskov U, Hviid L, Laursen SB and Jensenius JC (1992) The concentration of the C-type lectin, mannan-binding protein, in human plasma increases during an acute phase response. *Clin Exp Immunol* 90: 31-35
9. Madsen, H.O., Garred, P., Kurtzhals, J.A., Lamm, L.U., Ryder, L.P., Thiel, S., and Svejgaard, A. (1994) A new frequent allele is the missing link in the structural polymorphism of the human mannan-binding protein. *Immunogenetics* 40:37-44.
10. Summerfield JA, Ryder S, Sumiya M, Thursz M, Gorchein A, Monteil MA and Turner MW (1995) Mannose binding protein gene mutations associated with unusual and severe infections in adults. *Lancet* 345: 886-889
11. Garred P, Madsen HO, Hofmann B and Svejgaard A (1995) Increased frequency of homozygosity of abnormal mannan binding protein alleles in patients with suspected immunodeficiency. *Lancet* 346: 941-943
12. Summerfield JA, Sumiya M, Levin M and Turner MW (1997) Association of mutations in mannose-binding protein gene with childhood infection in consecutive hospital series. *BioMed J* 314: 1229-1232
13. van Emmerik, LC, Kuijper, EJ, Fijen, CAP, Dankert, J, and Thiel, S (1994) Binding of mannan-binding protein to various bacterial pathogens of meningitis. *Clin.Exp.Immunol.* 97:411-416.
14. Jack DL, Dodds AW, Anwar N, Ison CA, Law A, Frosch M, Turner MW and Klein NJ (1998) Activation of complement by Mannose-binding lectin on isogenic mutants of *Neisseria meningitidis* serogroup B. *J Immunol* 160: 1346-1353
15. Miller, M.E., Seals, J., Kaye, R., and Levitsky, L.C. (1968) A familial, plasma-associated defect of phagocytosis. A new case of recurrent bacterial infections. *The.Lancet* :60-63.
16. Super, M., Thiel, S., Lu, J., Levinsky, R.J., and Turner, M.W. (1989) Association of low levels of mannan-binding protein with a common defect of opsonisation. Lan*cet* 2:1236-1239.
17. Nielsen, S.L., Andersen, P.L., Koch, C., Jensenius, J.C., and Thiel, S. (1995) The level of the serum opsonin, mannan-binding protein in HIV-1 antibody-positive patients. *Clin. Exp. Immunol.* 100:219-222.
18.Christiansen, O.B., Kilpatrick, D.C., Souter, V., Varming, K., Thiel, S., Jensenius,J.C. (1999) Mannan-binding lectin deficiency is associated with unexplained recurrent miscarriage. Scand. J. Immunol., 49, 193-196
19. Garred, P, Harboe, M, Oettinger, T, Koch, C, and Svejgaard, A (1994) Dual role of mannanbinding protein in infections: Another case of heterosis ? *Eur.J.Immunogen.* 21:125-131.
20. Hoal-Van Helden EG, Epstein J, Victor TC, Hon D, Lewis LA, Beyers N, Zurakowski D, Ezekowitz AB, Van Helden PD (1999) Mannose-binding protein B allele confers protection against tuberculous meningitis. *Pediatr Res* 45:459-64
21. Fischer, PB, Ellerman-Eriksen, S, Thiel, S, Jensenius, JC, and Mogensen, SC (1994) Mannan-binding protein and conglutinin mediate enhancement of herpes simplex virus type-2 infection in mice. *Scand J Immunol* 39:439-445.
22. Valdimarsson H, Stefansson M, Vikingsdottir T, Arason GJ, Koch C, Thiel S and Jensenius JC (1998) Reconstitution of opsonizing activity by infusion of mannan-binding lectin (MBL) to
24. Pizzo, PA (1993), Management of fever in patients with cancer and treatment-induced neutropenia, N Eng J Med, 328, 1323-1332.
25. Aittoniemi, J., Miettinen, A., Laine, S., Sinisalo, M., Laippala, P., Vilpo, L, Vilpo, J. (1999), Opsonising immunoglobulins and mannan-binding lectin in chronic lymphocytic leukemia, Leuk Lymphoma Jul;34(34):3815
26. Lehrnbecher T, Venzon D, de Haas M, Chanock SJ, Kuhl J. (1999) Assessment of measuring circulating levels of interleukin6, interleukin8, Creactive protein, soluble Fc gamma receptor type III, and mannosebinding protein in febrile children with cancer and neutropenia. Clin Infect Dis, Aug;29(2):4149.
27. Lu, J., Thiel, S., Wiedemann, H., Timpl, R. & K.B.M. Reid (1990) Binding of the pentamer/hexamer forms of mannan-binding protein to zymosan activates the pro-enzyme C1r₂C1s₂ complex, of the classical pathway of complement without involvement of C1q. *J. Immunol.* **144**:2287-2294.
28. Sastry, K., Herman, G.A., Day, L., Deignan, E., Bruns, G., Morton, C.C. & R.A.B. Ezekowitz (1989) The human mannose-binding protein gene. *J. Exp. Med.* **170:**1175-1189.
29. Lipscombe, R.J., Sumiya, M., Summerfield, J.A. & M.W. Turner (1995) Distinct physicochemical characteristics of human mannose-binding protein expressed by individuals of differing genotype. *Immunology* **85**:660-667.

## Claims

1. Use of a composition comprising at least one collectin and/or ficolin subunit, such as mannan-binding lectin (MBL) subunit, or at least one collectin and/or ficolin oligomer comprising the collectin and/or ficolin subunit, such as a mannan-binding lectin (MBL) oligomer comprising the at least one mannan-binding lectin (MBL) subunit, in the manufacture of a medicament for prophylaxis and/or treatment of Severe Acute Respiratory Syndrome.

2. The use of claim 1, wherein the composition comprises at least one mannan-binding lectin (MBL) oligomer comprising the at least one mannan-binding lectin (MBL) subunit.

3. The use of claim 2, wherein said oligomer is preferably selected from the group of oligomers consisting of tetramers, pentamers and/or hexamers.

4. The use of claim 1, wherein the individual has a serum level of MBL in excess of 10 ng/ml serum.

5. The use of claim 1, wherein the individual has a serum level of MBL in excess of 50 ng/ml serum.

6. The use of any of claims 4 or 5, wherein the serum MBL level is the functional serum MBL level.

7. The use of claim 1, further comprising the manufacture of an antimicrobial medicament capable of attenuation and/or elimination a microbial species for obtaining a kit-of-parts.

8. The use of claim 7, further comprising the manufacture of an antibacterial medicament capable of bacterial attenuation and/or elimination for obtaining a kit-of-parts.

9. The use of claim 1, wherein the MBL subunit or the MBL oligomer is produced in a native host organism.

10. The use of claim 9, wherein the native host organism is a human cell natively expressing the MBL subunit or the MBL oligomer.

11. The use of claim 1, wherein the MBL subunit or MBL oligomer is produced by a host organism not natively expressing an MBL polypeptide.

12. The use of claim 1, wherein the MBL subunit or the MBL oligomer is produced by a method comprising at least one step of recombinant DNA technology in vitro.

13. The use of claim 11 or 12, wherein the production of the MBL subunit or the MBL oligomer is controlled by an expression control sequence not natively associated with MBL polypeptide expression.

14. The use of any of claims 9 to 13, wherein the MBL subunit or the MBL oligomer is isolated from the host organism.

15. The use of claim 14, wherein the MBL subunit or the MBL oligomer is isolated by a method comprising at least one step involving affinity chromatography.

16. The use of claim 13, wherein the affinity chromatography step is capable of isolating MBL tetramers, pentamers and/or hexamers from a composition further comprising additional MBL oligomers and/or MBL subunits.

17. The use of any of claims 11 to 16, wherein the MBL subunit and/or the MBL oligomer is free from any impurities naturally associated with the MBL when produced in a native host organism.

18. The use of claim 1, wherein the MBL subunit is a mammalian MBL subunit.

19. The use of claim 18, wherein the mammalian MBL subunit is a human MBL subunit.

20. The use of claim 1, wherein the medicament is to be administered to the individual prior to another treatment.

21. The use of any of the preceding claims, wherein the treatment is a prophylactic treatment.

22. The use of any of claims 1 to 21, wherein the medicament is a booster of MBL serum levels in an individual having MBL serum levels above a predetermined minimum MBL serum level of 10 ng/ml.

23. The use of claim 22, wherein the individual has MBL serum levels below a predetermined maximum MBL serum level of 500 ng/ml.

24. The use of claim 1 or 23, wherein the individual has serum levels of MBL in excess of 75 ng/ml.

25. The use of claim 1 or 23, wherein the individual has serum levels of MBL in excess of 100 ng/ml.

26. The use of claim 1 or 23, wherein the individual has serum levels of MBL in excess of 150 ng/ml.

27. The use of claim 1 or 24, wherein the individual has serum levels of MBL below 500 ng/ml.

28. The use of claim 1 or 24, wherein the individual has serum levels of MBL below 400 ng/ml.

29. The use of claim 1 or 24, wherein the individual has serum levels of MBL below 300 ng/ml.

30. The use of any of the preceding claims, wherein serum or plasma levels of MBL in the individual are determined by quantitative analysis.

31. The use of claim 30, wherein the analysis comprises at least one of ELISA, TRIFMA, RIA or nephelometry.

## Patentansprüche

1. Verwendung einer Zusammensetzung, die mindestens eine Collectin- und/oder eine Ficolin-Untereinheit, wie eine Mannan bindende Lektin-Untereinheit (MBL) umfasst, oder mindestens ein Collectin- und/oder ein Ficolin-Oligomer, das die Collectin- und/oder Ficolin-Untereinheit, wie ein Mannan bindendes Lektin (MBL) Oligomer umfasst, das die mindestens eine Mannan bindende Lektin (MBL)-Untereinheit umfasst, zur Herstellung eines Medikaments zur Prophylaxe und/oder Behandlung eines schweren akuten Atemweg-Syndroms.

2. Verwendung nach Anspruch 1, wobei die Zusammenfassung mindestens ein Mannan bindendes Lektin (MBL) Oligomer umfasst, das die mindestens eine Mannan bindende Lektin- (MBL) Untereinheit umfasst.

3. Verwendung nach Anspruch 2, wobei das Oligomer vorzugsweise ausgewählt ist aus der Gruppe von Oligomeren bestehend aus Tetrameren, Pentameren und/oder Hexameren.

4. Verwendung nach Anspruch 1, wobei das Individuum einen Serumspiegel von MBL im Überschuss von 10 ng/ml Serum aufweist.

5. Verwendung nach Anspruch 1, wobei das Individuum einen Serumspiegel von MBL im Überschuss von 50 ng/ml Serum aufweist.

6. Verwendung nach einem der Ansprüche 4 oder 5, wobei der Serumspiegel von MBL der funktionelle Serumspiegel von MBL ist.

7. Verwendung nach Anspruch 1, weiter umfassend die Herstellung eines antimikrobiellen Medikaments, um einen Kit-of Parts zu erhalten, das eine Mikrobenspezies verringern und/oder beseitigen kann.

8. Verwendung nach Anspruch 7, weiter umfassend die Herstellung eines antibakteriellen Medikaments, um einen Kit-of Parts zu erhalten, das Bakterien verringern und/oder beseitigen kann.

9. Verwendung nach Anspruch 1, wobei die MBL-Untereinheit oder das MBL-Oligomer in einem nativen Wirtsorganismus hergestellt wird.

10. Verwendung nach Anspruch 9, wobei der native Wirtsorganismus eine menschliche Zelle ist, die naturgemäß die MBL-Untereinheit oder das MBL-Oligomer exprimiert.

11. Verwendung nach Anspruch 1, wobei die MBL-Untereinheit oder das MBL-Oligomer durch einen Wirtsorganismus hergestellt wird, der ein MBL-Polypeptid nicht naturgemäß exprimiert.

12. Verwendung nach Anspruch 1, wobei die MBL-Untereinheit oder das MBL-Oligomer durch ein Verfahren hergestellt wird, das mindestens einen Schritt eines DNA-Rekombinationsverfahrens *in vitro* umfasst.

13. Verwendung nach Anspruch 11 oder 12, wobei die Herstellung der MBL-Untereinheit oder des MBL-Oligomers durch eine Expressionskontrollsequenz gesteuert wird, die naturgemäß nicht mit der MBL-Polypeptidexpression assoziiert ist.

14. Verwendung von einem der Ansprüche 9 bis 13, wobei die MBL-Untereinheit oder das MBL-Oligomer von dem Wirtsorganismus isoliert wird.

15. Verwendung nach Anspruch 14, wobei die MBL-Untereinheit oder das MBL-Oligomer durch ein Verfahren isoliert wird, das mindestens einen Schritt umfasst, der eine Affinitätschromatographie einschließt.

16. Verwendung nach Anspruch 13, wobei mit dem Schritt der Affinitätschromatographie MBL-Tetramere, Pentamere und/oder Hexamere aus einer Zusammensetzung isoliert werden können, die weitere zusätzliche MBL-Oligomere und/oder MBL-Untereinheiten umfasst.

17. Verwendung von einem der Ansprüche 11 bis 16, wobei die MBL-Untereinheit und/oder das MBL-Oligomer frei von irgendwelchen Unreinheiten ist, die naturgemäß mit dem MBL assoziiert sind, wenn es in einem nativen Wirtsorganismus hergestellt wird.

18. Verwendung nach Anspruch 1, wobei die MBL-Untereinheit eine MBL-Untereinheit eines Säugers ist.

19. Verwendung nach Anspruch 18, wobei die MBL-Untereinheit eines Säugers eine MBL-Untereinheit eines Menschen ist.

20. Verwendung nach Anspruch 1, wobei das Medikament dem Individuum vor einer anderen Behandlung zu verabreichen ist.

21. Verwendung nach einem der vorstehenden Ansprüche, wobei die Behandlung eine prophylaktische Behandlung ist.

22. Verwendung nach einem der Ansprüche 1 bis 21, wobei das Medikament ein Verstärker von MBL-Serumspiegeln in einem Individuum ist, das MBL-Serumspiegel aufweist, die über einem bestimmten minimalen MBL-Serumspiegel von 10 ng/ml liegen.

23. Verwendung nach Anspruch 22, wobei das Individuum MBL-Serumspiegel aufweist, die unter einem bestimmten maximalen MBL-Serumspiegel von 500 ng/ml liegen.

24. Verwendung nach Anspruch 1 oder 23, wobei das Individuum Serumspiegel von MBL in einem Überschuss von 75 ng/ml aufweist.

25. Verwendung nach Anspruch 1 oder 23, wobei das Individuum Serumspiegel von MBL im Überschuss von 100 ng/ml aufweist.

26. Verwendung nach Anspruch 1 oder 23, wobei das Individuum Serumspiegel von MBL im Überschuss von 150 ng/ml aufweist.

27. Verwendung nach Anspruch 1 oder 24, wobei das Individuum Serumspiegel von MBL von unter 500 ng/ml aufweist.

28. Verwendung nach Anspruch 1 oder 24, wobei das Individuum Serumspiegel von MBL von unter 400 ng/ml aufweist.

29. Verwendung nach Anspruch 1 oder 24, wobei das Individuum Serumspiegel von MBL von unter 300 ng/ml aufweist.

30. Verwendung nach einem der vorstehenden Ansprüche, wobei Serum- oder Plasmaspiegel von MBL in dem Individuum durch quantitative Analyse bestimmt werden.

31. Verwendung nach Anspruch 30, wobei die Analyse zumindest ELISA, TRIFMA, RIA oder Nephelometrie umfasst.

## Revendications

1. Utilisation d'une composition comprenant au moins une sous-unité de collectine et/ou de ficoline, comme une sous-unité de lectine liant le mannane (MBL), ou au moins un oligomère de collectine et/ou de ficoline comprenant la sous-unité de collectine et/ou de ficoline, comme un oligomère de lectine liant le mannane (MBL) comprenant la au moins une sous-unité de lectine liant le mannane (MBL), dans la fabrication d'un médicament pour la prophylaxie et/ou le traitement du syndrome respiratoire aigu sévère (SRAS).

2. Utilisation selon la revendication 1, dans laquelle la composition comprend au moins un oligomère de lectine se liant au mannane (MBL) comprenant la au moins une sous-unité de lectine se liant au mannane (MBL).

3. Utilisation selon la revendication 2, dans laquelle ledit oligomère est de préférence choisi dans le groupe d'oligomères consistant en les tétramères, les pentamères et/ou les hexamères.

4. Utilisation selon la revendication 1, dans laquelle le sujet a un niveau sérique de MBL dépassant 10 ng/ml de sérum.

5. Utilisation selon la revendication 1, dans laquelle le sujet a un niveau sérique de MBL dépassant 50 ng/ml de sérum.

6. Utilisation selon l'une quelconque des revendications 4 ou 5, dans laquelle le niveau sérique de MBL est le niveau sérique fonctionnel de MBL.

7. Utilisation selon la revendication 1, comprenant en outre la fabrication d'un médicament antimicrobien capable d'atténuer et/ou d'éliminer une espèce microbienne pour obtenir une trousse de pièces.

8. Utilisation selon la revendication 7, comprenant en outre la fabrication d'un médicament anti-bactérien capable d'atténuation et/ou l'élimination bactériennes pour obtenir une trousse de pièces.

9. Utilisation selon la revendication 1, dans laquelle la sous-unité de MBL ou l'oligomère de MBL sont produits dans un organisme hôte natif.

10. Utilisation selon la revendication 9, dans laquelle l'organisme hôte natif est une cellule humaine exprimant nativement la sous-unité de MBL ou l'oligomère de MBL.

11. Utilisation selon la revendication 1, dans laquelle la sous-unité de MBL ou l'oligomère de MBL sont produits par un organisme hôte n'exprimant pas nativement un polypeptide de MBL.

12. Utilisation selon la revendication 1, dans laquelle la sous-unité de MBL ou l'oligomère de MBL sont produits par un procédé comprenant au moins une étape de technologie de l'ADN recombinant *in vitro.*

13. Utilisation selon la revendication 11 ou 12, dans laquelle la production de la sous-unité de MBL ou de l'oligomère de MBL est contrôlée par une séquence de contrôle de l'expression non nativement associée à l'expression du polypeptide de MBL.

14. Utilisation selon l'une quelconque des revendications 9 à 13, dans laquelle la sous-unité de MBL ou l'oligomère de MBL sont isolés à partir de l'organisme hôte.

15. Utilisation selon la revendication 14, dans laquelle la sous-unité de MBL ou l'oligomère de MBL sont isolés par un procédé comprenant au moins une étape impliquant la chromatographie d'affinité.

16. Utilisation selon la revendication 13, dans laquelle l'étape de chromatographie d'affinité est capable d'isoler des tétramères, des pentamères et/ou des hexamères de MBL à partir d'une composition comprenant en outre d'autres oligomères et/ou sous-unités de MBL.

17. Utilisation selon l'une quelconque des revendications 11 à 16, dans laquelle la sous-unité de MBL et/ou l'oligomère de MBL sont exempts des impuretés naturellement associées à la MBL lorsqu'elle est produite dans un organisme hôte natif.

18. Utilisation selon la revendication 1, dans laquelle la sous-unité de MBL est une sous-unité de MBL de mammifère.

19. Utilisation selon la revendication 18, dans laquelle la sous-unité de MBL de mammifère est une sous-unité de MBL humaine.

20. Utilisation selon la revendication 1, dans laquelle le médicament doit être administré au sujet avant un autre traitement.

21. Utilisation selon l'une quelconque des revendications précédentes, dans laquelle le traitement est un traitement prophylactique.

22. Utilisation selon l'une quelconque des revendications 1 à 21, dans laquelle le médicament est un stimulateur des niveaux sériques de MBL chez un sujet ayant des niveaux de MBL sériques supérieurs à un niveau sérique de MBL minimal prédéterminé de 10 ng/ml.

23. Utilisation selon la revendication 22, dans laquelle le sujet a des niveaux sériques de MBL en deçà d'un niveau sérique de MBL maximal prédéterminé de 500 ng/ml.

24. Utilisation selon la revendication 1 à 23, dans laquelle le sujet a des niveaux sériques de MBL dépassant 75 ng/ml.

25. Utilisation selon la revendication 1 à 23, dans laquelle le sujet a des niveaux sériques de MBL dépassant 100 ng/ml.

26. Utilisation selon la revendication 1 à 23, dans laquelle le sujet a des niveaux sériques de MBL dépassant 150 ng/ml.

27. Utilisation selon la revendication 1 à 24, dans laquelle le sujet a des niveaux sériques de MBL inférieurs à 500 ng/ml.

28. Utilisation selon la revendication 1 à 24, dans laquelle le sujet a des niveaux sériques de MBL inférieurs à 400 ng/ml.

29. Utilisation selon la revendication 1 à 24, dans laquelle le sujet a des niveaux sériques de MBL inférieurs à 300 ng/ml.

30. Utilisation selon l'une quelconque des revendications précédentes, dans laquelle les niveaux sériques ou plasmatiques de MBL chez le sujet sont déterminés par analyse quantitative.

31. Utilisation selon la revendication 30, dans laquelle l'analyse comprend au moins une parmi ELISA, TRIFMA, RIA ou néphélométrie.
